# EUROPÄISCHE PATENTSCHRIFT

(11) **EP 1 053 718 B1**
(45) Veröffentlichungstag und Bekanntmachung des Hinweises auf die Patenterteilung: **09.07.2008**
(21) Anmeldenummer: 00810321.0
(22) Anmeldetag: 13.04.2000
(51) Int. Cl.: A61B 17/72

(54) **Verriegelungsnagel zur Versorgung von Femurschaftfrakturen**
Locking nail for the treatment of femoral shaft fractures
Clou de verrouillage pour le traitement des fractures de la tige fémorale

(30) Priorität: 12.05.1999 EP 99810422
(43) Veröffentlichungstag der Anmeldung: 22.11.2000
(73) Patentinhaber: Zimmer GmbH, 8404 Winterthur (CH)
(72) Erfinder: Adam, Michael, 3627 Heimberg (CH)
(74) Vertreter: Manitz, Finsterwald & Partner GbR

(56) Entgegenhaltungen:
- EP-A- 0 355 411
- EP-A- 0 528 128
- US-A- 2 518 019
- US-A- 4 135 507

## Beschreibung

Die Erfindung betrifft einen Verriegelungsnagel zur Versorgung von Femurschaftfrakturen, auch in Verbindung mit trochantären Femurfrakturen, gemäss dem Oberbegriff von Anspruch 1.

Aus der Druckschrift EP 0 528 128 ist ein Osteosynthesehilfsmittel zur Versorgung intertrochantärer oder subtrochantärer Femurfrakturen bekannt, welches als ein Verriegelungsnagel mit einem offenen Kleeblattprofil ausgestaltet ist. Der Verriegelungsnagel weist in seinem proximalen Abschnitt Schrägbohrungen zur Führung und Haltern einer Schenkelhalsschraube auf. Der Verriegelungsnagel weist an seinem distalen Abschnitt einen axialen Längsschlitz auf. Der Verriegelungsnagel weist zudem am Übergang zwischen einem proximalen Teilabschnitt und einem distalen Teilabschnitt einen Knick auf. Nachteilig an diesem bekannten Verriegelungsnagel ist die Tatsache, dass er schwierig in den Markraum einzuführen ist.

Es ist Aufgabe der vorliegenden Erfindung einen vorteilhafteren Verriegelungsnagel zur Versorgung von Femurschaftfrakturen zu schaffen.

Diese Aufgabe wird gelöst mit einem Verriegelungsnagel aufweisend die Merkmale von Anspruch 1. Die Unteransprüche 2 bis 13 betreffen weitere, vorteilhafte Ausgestaltungen des erfindungsgemässen Verriegelungsnagels. Die Aufgabe wird gelöst mit einem Verriegelungsnagel umfassend einen proximalen Nagelabschnitt sowie einen daran anschliessenden distalen Nagelabschnitt, wobei diese Nagelabschnitte Bohrungen zur Aufnahme von Knochenschrauben aufweisen, und wobei der distale Nagelabschnitt eine in einer anterior-posterior-Ebene verlaufende, im wesentlichen der Antekurvation des Femurs entsprechende Krümmung aufweist, und wobei der proximale Nagelabschnitt zumindest über einem Teilabschnitt eine in einer lateral-medial-Ebene verlaufende stetige Krümmung, mit konstantem Krümmungsradius, aufweist.

Unter dem Begriff _{"}stetige Krümmung" wird im weiteren verstanden, dass die Krümmung keine Stelle mit einer Unstetigkeit beziehungsweise keinen Knick aufweist, was mathematisch auch derart beschreibbar ist, dass die erste Ableitung des Krümmungsverlaufes keine Unstetigkeit beziehungsweise keine sprunghafte Veränderung aufweist.

In einer vorteilhaften Ausgestaltung weist der proximale Nagelabschnitt, ausgehend vom distalen Nagelabschnitt, einen Übergangsabschnitt und daran anschliessend einen Befestigungsabschnitt mit Bohrungen zur Aufnahme der Knochenschrauben auf, wobei der Übergangsabschnitt sowie der Befestigungsabschnitt eine durchgehende Krümmung mit konstantem Krümmungsradius aufweisen.

Der erfindungsgemässe Verriegelungsnagel ist insbesondere zur Versorgung von Femurschaftfrakturen, auch in Verbindung mit trochantären Femurfrakturen geeignet, wobei der Verriegelungsnagel, bedingt durch die interindividuell unterschiedliche anatomische Ausgestaltung des Femurs, mit Längen von etwa 300 mm bis 500 mm gefertigt wird. Der Verriegelungsnagel zeichnet sich durch einen guten mechanischen Halt in der Markhöhle aus und weist eine gute Verdrehsicherheit auf. Im unteren distalen Nagelabschnitt ist eine Querbohrung angeordnet, durch welche eine Knochenschraube eingeführt wird, um den Verriegelungsnagel axial und in Drehrichtung fest zu halten. Der proximale Nagelabschnitt weist vorzugsweise eine Schrägdurchbohrung auf, durch welche eine Schenkelhalsschraube einführbar ist.

Der erfindungsgemässe Verriegelungsnagel ist vorzugsweise als ein durchgängiges Hohlrohr, insbesondere als zylindrisches Hohlrohr ausgestaltet und besteht aus einem körperverträglichen, nichtkorrodierendem Material wie Titan oder einer Titanlegierung. In das durchgängige Hohlrohr wird vorzugsweise ein Führungsdraht eingeführt. Der Verriegelungsnagel könnte jedoch auch stangenförmig ausgestaltet sein und keinen in axialer Richtung verlaufenden, hohlen Innenraum aufweisen.

Ein Vorteil des erfindungsgemässen Verriegelungsnagels ist die Tatsache, dass dieser, selbst in einer Ausführungsform mit geringer Elastizität, ohne grössere Kraftaufwendung in den Markraum des Femurs einführbar ist. Die Ausführungsform mit geringer Elastizität gewährleistet einen sehr guten mechanischen Halt sowie eine gute Stützfunktion in der Markhöhle.

Der erfindungsgemässe Verriegelungsnagel ist derart ausgestaltet, dass dieser vorzugsweise ausgehend vom Trochanter major in den Femur beziehungsweise in dessen Markraum eingeführt wird, wobei die Einführstelle vorzugsweise leicht lateral bezüglich der Spitze des Trochanter major angeordnet ist. Da der Trochanter major, im Vergleich zu der bisher üblichen Einführstelle am Femur, näher an der Oberfläche der Haut angeordnet ist, weist das Einführen über den Trochanter major den Vorteil auf, dass eine kleinere Eröffnung des Operationsfeldes erforderlich ist, was insbesondere die Gefahr von Infektionen reduziert. Zudem ist der Trochanter major bei den während der Operation erforderlichen Tätigkeiten leichter zugänglich. Üblicherweise wurde ein Verriegelungsnagel im Bereich der Fossa piriformis in den Markraum eingeführt. An dieser Stelle befinden sich jedoch auch Blutgefässe usw., sodass das Einführen über den Trochanter major sicherer ist um Verletzungen dieser Blutgefässe zu vermeiden. In einer vorteilhaften Ausgestaltung weist der Verriegelungsnagel einen Befestigungsabschnitt mit einem relativ kleinen Querschnitt auf. Dies erlaubt einen Verriegelungsnagel mit einem gesamthaft kleinen Querschnitt zu bilden, was sich vorteilhaft auf das Einführen in den Markraum auswirkt und zudem eine nur kleine Eröffnung des Operationsfeldes erfordert.

Der aus der zitierten Druckschrift bekannte Verriegelungsnagel (worauf der Oberbegriff des Anspruchs 1 basiert) weist an der Übergangsstelle zwischen dem distalen und dem proximalen Nagelabschnitt eine Knickstelle auf und ist deshalb, trotz der durch den Längsschlitz im distalen Nagelabschnitt bewirkten Elastizität, schwierig in den Markraum einzuführen, da der Markraum einen gekrümmten Verlauf aufweist, und dieser Nagel an gewissen Stellen an der Markraumwand oder an der Spongiosa anliegt und nur mit grösserer Kraftaufwendung in den Markraum einführbar ist. Der erfindungsgemässe Verriegelungsnagel mit dem gekrümmt verlaufenden proximalen Nagelabschnitt weist den Vorteil auf, dass dieser den anatomischen Verlauf des Markraumes derart vorteilhaft berücksichtigt, dass dieser ohne grössere Kraftaufwendung einführbar ist. Im Gegensatz zu kurzen Verriegelungsnägeln muss der erfindungsgemässe, sehr lang ausgestaltete Verriegelungsnagel während dem Einführen in den Markraum um eine Teildrehung um dessen Längsachse gedreht werden. Der erfindungsgemässe Verriegelungsnagel weist die Eigenschaft auf, dass dieser sich während dem Einführen in den Markraum von selbst in die richtige Endlage dreht, da die Aussenform des Verriegelungsnagels bezüglich wesentlicher Aspekte dem Verlauf des Markraumes nachgebildet ist. Obwohl die Möglichkeit in EP 0 528 128 erwähnt wird, den Nagel entsprechend der physiologischen Antekurvation des Femurs gebogen herzustellen, hat die darin beschriebene Ausführung einen geradlinigen distalen Abschnitt, und der aus der zitierten Druckschrift bekannte Verriegelungsnagel weist an der Übergangsstelle zwischen dem proximalen und distalen Nagelabschnitt einen Knick auf, was ein selbsttätiges Drehen während dem Einführen in den Markraum verhindert. US-A-4 135 507 offenbart einen Marknagel der sowohl proximal als auch distal eine stettige Krümmung aufweist, derer Radius aber nicht konstant ist. Der aus EP 0 528 128 bekannte Verriegelungsnagel weist den weiteren Nachteil auf, dass der im Markraum eingeführte Nagel nur bedingt drehbar ist, da Material in den Längsschlitz hineinragt und während dem Drehen ein Hindernis ausbildet. Da der erfindungsgemässe Verriegelungsnagel nicht unbedingt einen Längsschlitz bedarf, ist er zudem kostengünstiger herstellbar.

Der erfindungsgemässe Verriegelungsnagel kann eine geringe Elastizität aufweisen und trotzdem sicher und ohne grössere Kraftaufwendung in den Markraum eingeführt werden. Durch die erfindungsgemässe Ausgestaltung des Verriegelungsnagels wird während dessen Einführen in den Markraum eine grössere, bezüglich dem Verlauf des Femurs etwa radial nach Aussen gerichtete, insbesondere die Kortikals des Femurs schwächende oder zerstörende Kraft vermieden. Dadurch wird zudem ermöglicht, dass der Femur in seiner anatomisch richtigen Lage zusammengehalten wird. Es ist bekannt, dass ein ungünstig ausgestalteter Verriegelungsnagel bewirken kann, dass sich der Frakturen aufweisende Femur dem Verlauf des Verriegelungsnagels anpasst, was zur Folge hat, dass der fixierte Femur eine Fehlstellung beziehungsweise eine Abweichung von der Normalstellung aufweist.

Ausführungsbeispiele des erfindungsgemässen Verriegelungsnagels werden nachfolgend anhand von Zeichnungen näher erläutert. Es zeigen:
Fig. 1 eine Seitenansicht des Verriegelungsnagels aus Richtung B;
Fig. 2 eine Vorderansicht des Verriegelungsnagels aus Richtung A;
Fig. 3 eine Vorderansicht des leicht um dessen Achse gedrehten Verriegelungsnagels aus Richtung C, teilweise im Schnitt;
Fig. 4 eine Detailansicht des proximalen Nagelabschnittes aus Richtung D;
Fig. 5 eine Detailansicht des Verriegelungsnagels aus Richtung E;
Fig. 6 einen Querschnitt durch einen geschlitzten Verriegelungsnagel;
Fig. 7 eine Detailansicht des Befestigungsabschnittes aus Richtung F.

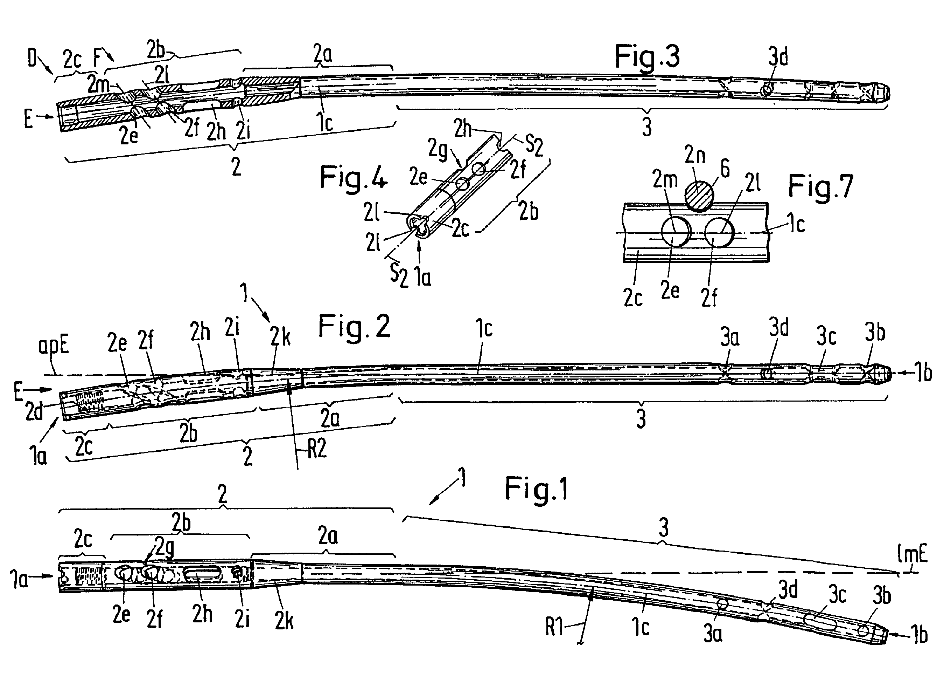

Der Verriegelungsnagel 1 gemäss Fig. 1 ist einstückig ausgebildet und besteht aus einem proximalen Nagelabschnitt 2 sowie einem daran anschliessenden distalen Nagelabschnitt 3. Ausgehend vom proximalen Ende 1a des Verriegelungsnagels 1 umfasst der proximale Nagelabschnitt 2 einen geradlinig verlaufenden Gewindeabschnitt 2c mit einem Innengewinde 2d, daran anschliessend einen Befestigungsabschnitt 2b, welcher eine Querbohrung 2i, eine ovale Querbohrung 2h, sowie zwei Schrägbohrungen 2e, 2f aufweist, sowie daran anschliessend einen Übergangsabschnitt 2a, welcher sich konisch verjüngend zum distalen Nagelabschnitt 3 erstreckt. Die Oberfläche des Befestigungsabschnittes 2b weist eine parallel zu den Schrägbohrungen 2e, 2f verlaufende Nut 2g auf. Abhängig von der Fraktur lassen sich in die Schrägbohrungen 2e, 2f und/oder die Nut 2g Knochenschrauben einführen um den Femurkopf zu sichern. Dazu wird vorzugsweise ein Zielgerät oder ein Einschlagwerkzeug verwendet, das bei in den Femur eingesetztem Verriegelungsnagel 1 vorübergehend in das Innengewinde 2d des Gewindeabschnittes 2c angeschraubt wird, um die Knochenschrauben genau geführt einzuschrauben.

Anschliessend an den Übergangsabschnitt 2a erstreckt sich der distale Nagelabschnitt 3 bis zum distalen Nagelende 1b. Zwischen dem proximalen Nagelende 1a sowie dem distalen Nagelende 1b ist die in der Mitte des Verriegelungsnagels 1 verlaufende Mittelllinie 1c dargestellt. Die anterior-posterior-Ebene apE verläuft entsprechender der Betrachtungsebene gemäss Fig. 1. Der distale Nagelabschnitt 3 weist eine in dieser anterior-posterior-Ebene apE verlaufende Krümmung auf, welche im wesentlichen entsprechend der Antekurvation des Femurs ausgestaltet ist. Vorzugsweise ist die Krümmung des distalen Nagelabschnittes 3 beziehungsweise dessen Mittellinie 1c als Kreissegment ausgestaltet, wobei der Krümmungsradius R1, abhängig von der anatomischen Gestalt des Femurs, beispielsweise 2 Meter beträgt. Im distalen Nagelabschnitt 3 sind Querbohrungen 3a, 3b, 3d sowie eine ovale Querbohrung 3c zur Aufnahme von Knochenschrauben angebracht.

In Fig. 1 ist zudem die senkrecht zur Betrachtungsebene bzw. zur anterior-posterior-Ebene apE verlaufende lateral-medial-Ebene ImE dargestellt. Die lateral-medial-Ebene ImE verläuft entsprechender der Betrachtungsebene gemäss Fig. 2. In Fig. 2 ist zudem die senkrecht zur Betrachtungsebene bzw. zur lateral-medial-Ebene ImE verlaufende anterior-posterior-Ebene apE dargestellt.

Die Vorderansicht des Verriegelungsnagels 1 aus Richtung A gemäss Fig. 2 zeigt den aus dieser Sicht in der lateral-medial-Ebene ImE geradlinig verlaufenden distalen Nagelabschnitt 3, an welchen die beiden, eine gemeinsame Krümmung mit konstantem Krümmungsradius R2 aufweisenden Übergangsabschnitt 2a sowie Befestigungsabschnitt 2b anschliessen. Der anschliessende Gewindeabschnitt 2c ist im dargestellten Ausführungsbeispiel geradlinig verlaufend ausgestaltet, könnte jedoch auch gekrümmt verlaufend ausgestaltet sein und insbesondere denselben Krümmungsradius R2 aufweisen. Der Teilabschnitt 2a, 2b beziehungsweise die Mittellinie 1c, welche in der lateral-medial-Ebene ImE verläuft, weist einen konstanten Krümmungsradius R2 auf, und erstreckt sich in axialer Richtung zumindest über etwa einen Viertel der Gesamtlänge des Verriegelungsnagels 1, und vorzugsweise etwa über einen Drittel der Gesamtlänge.

Die in Fig. 4 dargestellte Detailansicht aus Richtung D zeigt einen Teil des proximalen Nagelabschnittes 2, nämlich den Befestigungsabschnitt 2b sowie den Gewindeabschnitt 2c. Im Befestigungsabschnitt 2b sind die Öffnungen der ersten und zweiten Schrägbohrung 2e, 2f, die Kerbe 2g sowie ein Teil der ovalen Querbohrung 2h ersichtlich. Zudem weist der proximale Nagelabschnitt 2 am proximalen Nagelende 1a zwei Kerben 2l auf. Die Kerben 2l, die Schrägbohrungen 2e, 2f sowie die ovale Querbohrung 2h verlaufen alle in derselben Ebene S2. Wie aus Fig. 3 ersichtlich verlaufen die Mittellinien 2m, 2l der Schrägbohrungen 2e, 2f parallel zueinander, wobei in Fig. 4 zudem die Kerbe 2g parallel zu den Schrägbohrungen 2e, 2f verläuft. In weiteren Ausführungsbeispielen könnten die Schrägbohrungen 2e, 2f auch nicht parallel zueinander verlaufen.

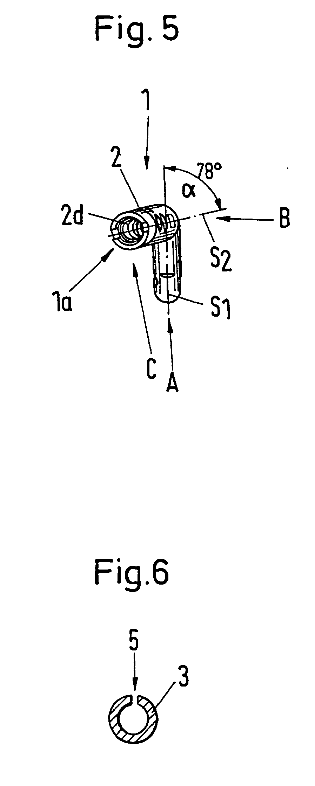

Fig. 7 zeigt eine Aufsicht aus Richtung F auf einen Ausschnitt des Befestigungsabschnittes 2b mit Schrägbohrungen 2e,2f und Kerbe 2g, wobei die Richtung F parallel zu den Mittellinien 2m, 2l verläuft. In der ventral angeordneten Kerbe 2g ist eine Schraube 6 mit Mittellinie 2n angeordnet. Im dargestellten Ausführungsbeispiel sind die Mittellinien 2m, 2l, 2n unter Ausbildung eines gleichschenkligen Dreiecks beabstandet, wobei die Mittellinien 2m, 2l durch die Mittellinie 1c des Verriegelungsnagels 1 verlaufen. Der Abstand zwischen den Mittellinien 2l, 2n sowie den Mittellinien 2m, 2n beträgt beispielsweise 8 mm. Der proximale Nagelabschnitt 2 wird durch eine, zwei oder drei Schrauben 6, welche durch die zwei Schrägbohrungen 2e, 2f sowie der Kerbe 2g verlaufend angeordnet werden, fest mit dem Femur verbunden. Diese Anordnung von zwei Schrägbohrungen 2e, 2f sowie der Kerbe 2g weist den Vorteil auf, dass der Durchmesser des proximalen Nagelabschnittes 2 relativ dünn ausgestaltet werden kann und trotzdem eine sichere Verankerung des Verriegelungsnagels 1 mittels Knochenschrauben 6 möglich ist. Der Verriegelungsnagel 1 kann beispielsweise derart ausgestaltet sein, dass der proximale Nagelabschnitt 2 einen Durchmesser zwischen 13 und 15 mm aufweist, und der distale Nagelabschnitt 3 einen Durchmesser zwischen 9 und 13 mm aufweist. Ein derart dünn ausgestalteter Verriegelungsnagel 1 weist den Vorteil auf, dass es zu dessen Implantation einer nur kleinen Eröffnung des Operationsfeldes bedarf. Bedingt durch die kleine erforderliche Hautöffnung besteht somit während dem Implantieren auch eine kleinere Infektionsgefahr. Der Befestigungsabschnitt 2b mit Schrägbohrungen 2e, 2f sowie ventral angeordneter Kerbe 2g, insbesondere gemäss der in Fig. 4 und Fig. 7 dargestellten Ausführungsform, könnte auch losgelöst von den übrigen Teilen 2c,2a und dem distalen Nagelabschnitt 3 in einem anders ausgestalteten Verriegelungsnagel verwendet werden. Ein derartiger Befestigungsabschnitt 2b könnte in axialer Richtung auch geradlinig beziehungsweise krümmungslos verlaufend ausgestaltet sein. Fig. 5 zeigt eine Frontansicht des proximalen Nagelendes 1a aus Richtung E. Ausgehend von proximalen Nagelende 1a verläuft der Verriegelungsnagel 1 entlang des proximalen Nagelabschnittes 2 in einer Linkskurve, und verläuft nach dem Übergang in den distalen Nagelabschnitt 3 in einer nach unten weisenden Kurve. Eine in Betrachtungsrichtung senkrecht durch den distalen Nagelabschnitt 3 verlaufende Ebene S1 und die durch die Kerben 2l, die Schrägbohrungen 2e, 2f sowie die ovale Querbohrung 2h verlaufende Ebene S2 schneiden sich unter einem Winkel von 78 Grad. Der proximale Nagelabschnitt 2 weist somit gegenüber dem distalen Nagelabschnitt 1 eine Antetorsion von 78 Grad auf. Diese Antetorsion ist erforderlich, damit die Schrägbohrungen 2e, 2f bzw. die Querbohrung 2h bei im Femur eingesetztem Verriegelungsnagel 1 in Richtung des Schenkelhalses bzw. des Femurkopfes verlaufen. Die Antetorsion beträgt, abhängig von der anatomischen Gestalt des Femurs, vorzugsweise zwischen 75 Grad und 80 Grad. Der deutsche Ausdruck _{"}Antetorsion" wird im Englischen als _{"}Anteversion" bezeichnet.

Fig. 3 zeigt den Verriegelungsnagel 1 aus der Blickrichtung C, welche senkrecht zur Ebene S2 verläuft. Gegenüber der Darstellung gemäss Fig. 2 aus Blickrichtung A ist der Verriegelungsnagel 1 gemäss Fig. 3 um 12 Grad um dessen Längsachse gedreht dargestellt. Die Schnittebene durch den proximalen Nagelabschnitt 2 entspricht der Ebene S2 und verläuft parallel zur Betrachtungsebene.

Fig. 6 zeigt einen Schnitt durch ein weiteres Ausführungsbeispiel eines distalen Nagelabschnittes 3, welcher einen in dessen Längsrichtung verlaufender Spalt 5 aufweist, um dem Nagelabschnitt 3 eine höhere Elastizität zu verleihen.

Der in den Figuren 1 bis 5 dargestellte Verriegelungsnagel 1 wird für einen linken Femur bestimmt. Auf Grund der physiologischen Antekurvation und Antetorsion des Femurs ist eine Rechts- und eine Links-Version des Verriegelungsnagels 1 erforderlich.

## Patentansprüche

1. Verriegelungsnagel zur Versorgung von Femurschaftfrakturen, auch in Verbindung mit trochantären Femurfrakturen, umfassend einen proximalen Nagelabschnitt (2) sowie einen daran anschliessenden distalen Nagelabschnitt (3), wobei die Nagelabschnitte (2,3) Bohrungen (2e,2f,2h,2i, 3a, 3b, 3c) zur Aufnahme von Knochenschrauben aufweisen, und wobei der distale Nagelabschnitt (3) eine in einer anterior-posterior-Ebene (apE) verlaufende, im wesentlichen der Antekurvation des Femurs entsprechende Krümmung aufweist, **dadurch gekennzeichnet, dass** der proximale Nagelabschnitt (2) zumindest über einem Teilabschnitt (2a,2b) eine in einer lateral-medial-Ebene (ImE) verlaufende, stetige Krümmung mit konstantem Krümmungsradius (R2) aufweist.

2. Verriegelungsnagel (1) nach Anspruch 1, **dadurch gekennzeichnet, dass** sich der Teilabschnitt (2a,2b) aufweisend eine stetige Krümmung in axialer Richtung zumindest über etwa einen Viertel der Gesamtlänge des Verriegelungsnagels (1) erstreckt, vorzugsweise über etwa einen Drittel der Gesamtlänge.

3. Verriegelungsnagel (1) nach einem der vorhergehenden Ansprüche, **dadurch gekennzeichnet, dass** der proximale Nagelabschnitt (2), ausgehend vom distalen Nagelabschnitt (3), aus einem Übergangsabschnitt (2a), einem daran anschliessenden Befestigungsabschnitt (2b) sowie einem daran anschliessenden Gewindeabschnitt (2c) besteht, dass der Befestigungsabschnitt (2b) Bohrungen (2e,2f,2h,2i) aufweist, und dass zumindest der Übergangsabschnitt (2a) sowie der Befestigungsabschnitt (2b) eine gemeinsame Krümmung mit konstantem Krümmungsradius (R2) aufweisen.

4. Verriegelungsnagel (1) nach einem der vorhergehenden Ansprüche, **dadurch gekennzeichnet, dass** der proximale Nagelabschnitt (2) zumindest eine Schrägbohrung (2e,2f) aufweist.

5. Verriegelungsnagel (1) nach Anspruch 4, **dadurch gekennzeichnet, dass** der proximale Nagelabschnitt (2) an dessen Oberfläche zumindest eine, im wesentlichen parallel zu der zumindest einen Schrägbohrung (2e,2f) verlaufende Kerbe (2g) aufweist.

6. Verriegelungsnagel (1) nach Anspruch 5, **dadurch gekennzeichnet, dass** die Kerbe (2g) ventral verlaufend angeordnet ist.

7. Verriegelungsnagel (1) nach einem der Ansprüche 4 bis 6, **dadurch gekennzeichnet, dass** die Schrägbohrungen (2e, 2f) parallel verlaufen.

8. Verriegelungsnagel (1) nach einem der Ansprüche 4 bis 7, **dadurch gekennzeichnet, dass** zwei Schrägbohrungen (2e, 2f) sowie eine Kerbe (2g) parallel verlaufend und insbesondere unter Ausbildung eines gleichschenkligen oder gleichseitigen Dreiecks angeordnet sind.

9. Verriegelungsnagel (1) nach einem der Ansprüche 3 bis 8, **dadurch gekennzeichnet, dass** der Gewindeabschnitt (2c) geradlinig verlaufend ausgestaltet ist und zum proximalen Nagelende (1a) ausläuft, wobei der Gewindeabschnitt (2c) ein Innengewinde (2d) zur Aufnahme eines Zielgerätes oder eines Ein- oder Ausschlagwerkzeuges aufweist.

10. Verriegelungsnagel (1) nach einem der vorhergehenden Ansprüche, **dadurch gekennzeichnet, dass** die Bohrungen (2e, 2f, 2h, 2i) derart im proximalen Nagelabschnitt (2) verlaufend angeordnet sind, dass deren Achsen bezüglich dem distalen Nagelabschnitt (3) eine Antetorsion (α) von 75 Grad bis 80 Grad, und insbesondere eine Antetorsion (α) von 78 Grad aufweist.

11. Verriegelungsnagel (1) nach einem der Ansprüche 3 bis 10, **dadurch gekennzeichnet, dass** der Übergangsabschnitt (2a) sich konisch verjüngend vom Befestigungsabschnitt (2b) zum distalen Nagelabschnitt (3) übergeht.

12. Verriegelungsnagel (1) nach einem der vorhergehenden Ansprüche, **dadurch gekennzeichnet, dass** dieser als ein Hohlrohr ausgestaltet ist.

13. Verriegelungsnagel (1) nach einem der vorhergehenden Ansprüche, **dadurch gekennzeichnet, dass** das Hohlrohr im distalen Nagelabschnitt (3) einen in axialer Richtung verlaufenden Schlitz (5) aufweist.

## Claims

1. A locking nail for the treatment of femur shaft fractures, also in connection with trochanteric femur fractures, comprising a proximal nail section (2) and a distal nail section (3) adjoining the latter, with the nail sections (2, 3) having bores (2e, 2f, 2h, 2i, 3a, 3b, 3c) for the reception of bone screws, and with the distal nail section (3) having a curvature extending in an anterior-posterior plane (apE) and corresponding substantially to the antecurvature of the femur, **characterised in that** the proximal nail section (2) has, at least over a partial section (2a, 2b), a continuous curvature with a constant radius of curvature (R2), extending in a lateral-medial plane (lmE).

2. A locking nail (1) in accordance with claim 1, **characterised in that** the partial section (2a, 2b) having a continuous curvature in the axial direction extends at least over approximately a quarter of the total length of the locking nail (1), preferably over approximately a third of the total length.

3. A locking nail (1) in accordance with any one of the preceding claims, **characterised in that** the proximal nail section (2), starting from the distal nail section (3), consists of a transition section (2a), a securing section (2b) adjoining the latter and a thread section (2c) adjoining the latter; **in that** the securing section (2b) has bores (2e, 2f, 2h, 2i); and **in that** at least the transition section (2a) and the securing section (2b) have a common curvature with a constant radius of curvature (R2).

4. A locking nail (1) in accordance with any one of the preceding claims, **characterised in that** the proximal nail section (2) has at least one inclined bore (2e, 2f).

5. A locking nail (1) in accordance with claim 4, **characterised in that** the proximal nail section (2) has at its surface at least one notch (2g) which extends substantially parallel to the at least one inclined bore (2e, 2f).

6. A locking nail (1) in accordance with claim 5, **characterised in that** the notch (2g) is arranged to extend ventrally.

7. A locking nail (1) in accordance with any one of the claims 4 to 6, **characterised in that** the inclined bores (2e, 2f) extend parallel to one another.

8. A locking nail (1) in accordance with any one of the claims 4 to 7, **characterised in that** two inclined bores (2e, 2f) and a notch (2g) are arranged to extend parallel to one another and in particular while forming an isosceles or equilateral triangle.

9. A locking nail (1) in accordance with any one of the claims 3 to 8, **characterised in that** the thread section (2c) is designed to extend in a straight line and runs out toward the proximal nail end (1a), with the thread section (2c) having an internal thread (2d) for the reception of an aiming device or a tool for hammering in or out.

10. A locking nail (1) in accordance with any one of the preceding claims, **characterised in that** the bores (2e, 2f, 2h, 2i) are arranged to extend in the proximal nail section (2) in such a manner that their axes have an anteversion (α) of 75 degrees to 80 degrees, and in particular an anteversion (α) of 78 degrees, relative to the distal nail section (3).

11. A locking nail (1) in accordance with any one of the claims 3 to 10, **characterised in that** the transition section (2a) conically convergingly merges from the securing section (2b) toward the distal nail section (3).

12. A locking nail (1) in accordance with any one of the preceding claims, **characterised in that** the latter is designed as a hollow tube.

13. A locking nail (1) in accordance with any one of the preceding claims, **characterised in that** the hollow tube has a slit (5) extending in the axial direction in the distal nail section (3).

## Revendications

1. Clou de verrouillage pour le traitement de fracture de la tige fémorale, également en association avec des fractures fémorales du trochanter, comprenant un tronçon de clou proximal (2) ainsi qu'un tronçon de clou distal (3) qui y fait suite, dans lequel les tronçons de clou (2, 3) présentent des perçages (2e, 2f, 2h, 2i, 3a, 3b, 3c) pour recevoir des vis à os, et dans lequel le tronçon de clou distal (3) présente une courbure s'étendant dans un plan antérieur-postérieur et correspondant essentiellement à la courbure antérieure du fémur, **caractérisé en ce que** le tronçon de clou proximal (2) présente, au moins sur un tronçon partiel (2a, 2b), une courbure permanente, s'étendant dans un plan latéral-médial, avec un rayon de courbure constant (R2).

2. Clou de verrouillage (1) selon la revendication 1, **caractérisé en ce que** le tronçon partiel (2a, 2b) qui présente une courbure permanente, s'étend en direction axiale au moins sur approximativement un quart de la longueur totale du clou de verrouillage (1), de préférence sur approximativement un tiers de la longueur totale.

3. Clou de verrouillage (1) selon l'une des revendications précédentes, **caractérisé en ce que** le tronçon de clou proximal (2) est constitué, en partant du tronçon de clou distal (3), d'un tronçon de transition (2a), d'un tronçon de fixation (2b) qui y fait suite, et d'un tronçon à pas de vis (2c) qui y fait suite, **en ce que** le tronçon de fixation (2b) comprend des perçages (2e, 2f, 2h, 2i), et **en ce que** le tronçon de transition (2a) et le tronçon de fixation (2b) au moins présentent une courbure commune avec un rayon de courbure constant (R2).

4. Clou de verrouillage (1) selon l'une des revendications précédentes, **caractérisé en ce que** le tronçon de clou proximal (2) présente au moins un perçage oblique (2e, 2f).

5. Clou de verrouillage (1) selon la revendication 4, **caractérisé en ce que** le tronçon de clou proximal (2) présente sur sa surface au moins une entaille (2g) s'étendant au essentiellement parallèlement audit au moins un perçage oblique (2e, 2f).

6. Clou de verrouillage (1) selon la revendication 5, **caractérisé en ce que** l'entaille (2g) est agencée de manière à s'étendre en situation ventrale.

7. Clou de verrouillage (1) selon l'une des revendications 4 à 6, **caractérisé en ce que** les perçages obliques (2e, 2f) s'étendent en parallèle.

8. Clou de verrouillage (1) selon l'une des revendications 4 à 7, **caractérisé en ce que** deux perçages obliques (2e, 2f) ainsi qu'une entaille (2g) s'étendent parallèlement et sont agencés en particulier en formant un triangle isocèle ou équilatéral.

9. Clou de verrouillage (1) selon l'une des revendications 3 à 8, **caractérisé en ce que** le tronçon à pas de vis (2c) est conçu de manière à s'étendre en ligne droite et se termine vers l'extrémité de clou proximal (1a), dans lequel le tronçon à pas de vis (2c) comprend un taraudage (2d) pour recevoir un dispositif de ciblage ou un outil à frapper pour l'entrée ou la sortie.

10. Clou de verrouillage (1) selon l'une des revendications précédentes, **caractérisé en ce que** les perçages (2e, 2f, 2h, 2i) sont agencés en s'étendant dans le tronçon de clou proximal (2) de telle manière que leurs axes présentent, par rapport au tronçon de clou distal (3), une torsion antérieure (α) de 75° à 80°, et en particulier une torsion antérieure (α) de 78°.

11. Clou de verrouillage (1) selon l'une des revendications 3 à 10, **caractérisé en ce que** le tronçon de transition (2a) va en se rétrécissant sous forme conique dans sa transition depuis le tronçon de fixation (2b) vers le tronçon de clou distal (3).

12. Clou de verrouillage (1) selon l'une des revendications précédentes, **caractérisé en ce que** celui-ci est conçu sous la forme de tube creux.

13. Clou de verrouillage (1) selon l'une des revendications précédentes, **caractérisé en ce que** le tube le creux présente, dans le tronçon de clou distal (3), une fente (5) s'étendant en direction axiale.
